# EUROPEAN PATENT APPLICATION

(11) **EP 3 769 815 A1**
(43) Date of publication of application: **27.01.2021**
(21) Application number: 19187930.3
(22) Date of filing: 23.07.2019
(51) Int. Cl.: A61P 31/10, C07D 311/30

(54) **ANTIMYCOTIC**

(71) Applicant: Karl-Franzens-Universität Graz, 8010 Graz (AT)
(72) Inventor: Bauer, Maria, 8010 Graz (AT); Kainz, Katharina, 8010 Graz (AT); Zimmermann, Andreas, 8010 Graz (AT); Carmona-Gutierrez, Didac, 8010 Graz (AT); Madeo, Frank, 8010 Graz (AT)
(74) Representative: Pföstl, Andreas

(57) **Abstract**

The present invention relates to methods and means for inhibiting or preventing the growth of non-filamentous fungal cells with at least one flavone of formula (I) 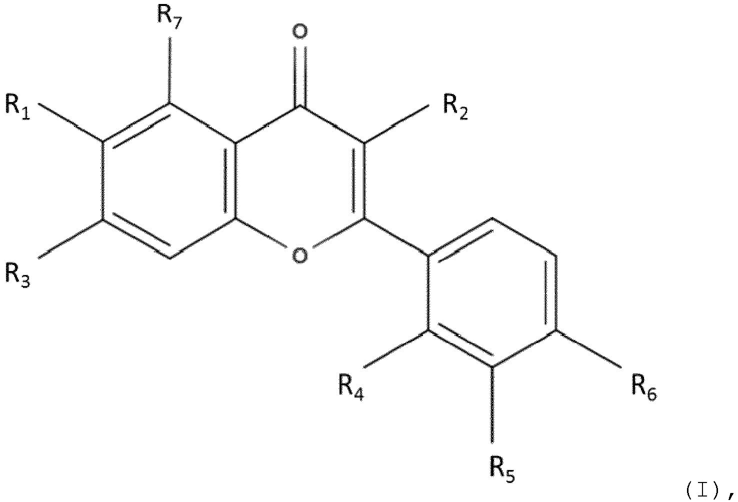 wherein R₁, R₂, R₃, R₄, R₅, R₆ and R₇ are independently from each other H or OH.

## Description

### TECHNICAL FIELD

The present invention relates to the field of antimycotics.

### BACKGROUND ART

Fungal infections are rapidly developing into an increasing medical and socioeconomic problem. Both the number of infections as well as resulting deaths are increasing rapidly, so pandemic proportions may arise in the near future. Due to the eukaryotic nature of the fungal cell and the resulting close relationship to human cells, makes the search for new antifungal agents even more difficult.

Currently, there are only a very limited number of antimycotics on the market, which, however, sometimes cause serious side effects or are not as efficient as required. The effectiveness is increasingly limited as the incidence of resistance to it increases rapidly. Therefore, the search for new antifungal active substances is of major importance.

Besides the search of novel antimycotics, the search for so-called "potentiators", i.e. substances that significantly increase the antifungal effect of antimycotics, has become increasingly important in recent years. Such substances may be able not only to increase the effect of antimycotics on fungal cells but also to broaden the effect of antimycotics on fungal cells whose viability cannot be effectively reduced using such antimycotics.

Hence, it is an object of the present invention to provide methods, compounds and combination of compounds to effectively inhibit the growth of fungal cells *ex vivo* as well as *in vivo* and to affect their viability. Another object of the present invention is to provide compounds that are able to enhance the efficacy of antimycotics.

### SUMMARY OF THE INVENTION

Thus, the present invention relates to the use of a flavone of formula (I) 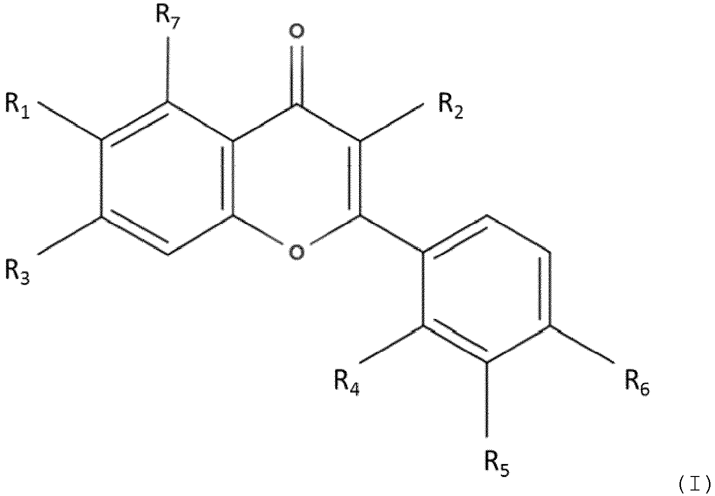 for inhibiting or preventing the growth of a non-filamentous fungal cell, wherein R₁, R₂, R₃, R₄, R₅, R₆ and R₇ are independently from each other H or OH.

Flavonoids are phytochemicals that are ubiquitous in plants and therefore also present in human food. Flavones, a class of flavonoids, have a 2-phenylchromen-4-one (2-phenyl-1-benzopyran-4-one) backbone and are commonly present in the food supply, mainly from spices, and red-purple fruits and vegetables. It turned surprisingly out that flavones having or consisting of formula (I) are able to inhibit the growth of non-filamentous fungal cells so that the viability and the number of fungal cells is significantly decreased. These antimycotic properties may not only be used for decreasing the number of viable fungal cells but also to prevent that fungal cells grow or reproduce.

The flavones of the present invention induce cell death in fungal cells. This was exemplarily confirmed in the pathogenic yeast *Candida albicans* and *Candida glabrata.* Surprisingly, the flavones of the present invention can be used in the treatment and in the control of either planktonic fungal cells or fungal cells being part of a biofilm. Treatment of both planktonic cells and biofilms of *C. albicans* and/or *C. glabrata* with the flavones of the present invention resulted in a decreased proliferation of the pathogens as well as inhibition of biofilm formation. The antifungal potential of the flavones of the present invention could be demonstrated *in vivo* using a *Caenorhabditis elegans* infection model. In particular di-substituted flavones, like 3,6-dihydroxyflavone (DHF), turned out to show good antimycotic properties.

Another aspect of the present invention relates to a composition comprising at least one flavone of formula (I) and at least one further antimycotic compound, wherein R₁, R₂, R₃, R₄, R₅, R₆ and R₇ are independently from each other H or OH.

Besides the antimycotic properties of the flavones of the present invention it turned surprisingly out that these flavones enhance the antifungal activity of other antimycotics.

Yet another aspect of the present invention relates to a composition according to the present invention or at least one flavone according to the present invention for the use in the treatment of fungal infections, preferably caused by non-filamentous fungal cells, in a human or animal, preferably mammalian, subject.

A further aspect of the present invention relates to a method for inhibiting or preventing the growth of fungal cells, preferably of non-filamentous fungal cells, comprising the step of contacting fungal cells with at least one flavone and/or a composition of the present invention.

### DESCRIPTION OF THE FIGURES

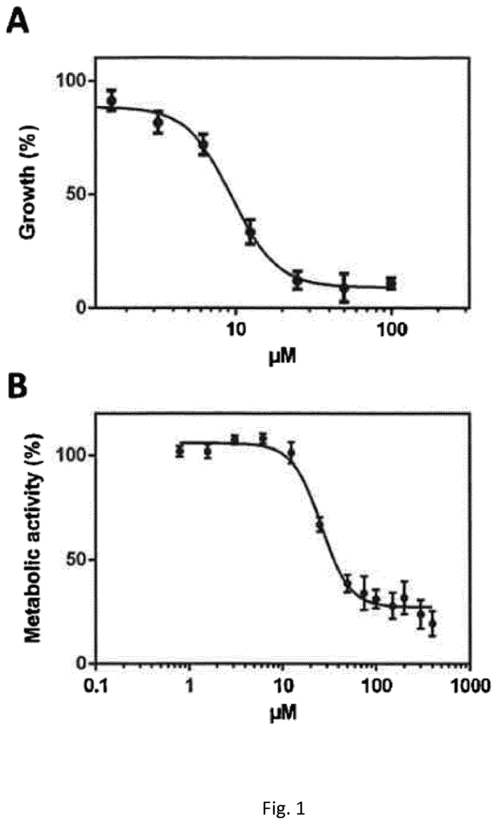

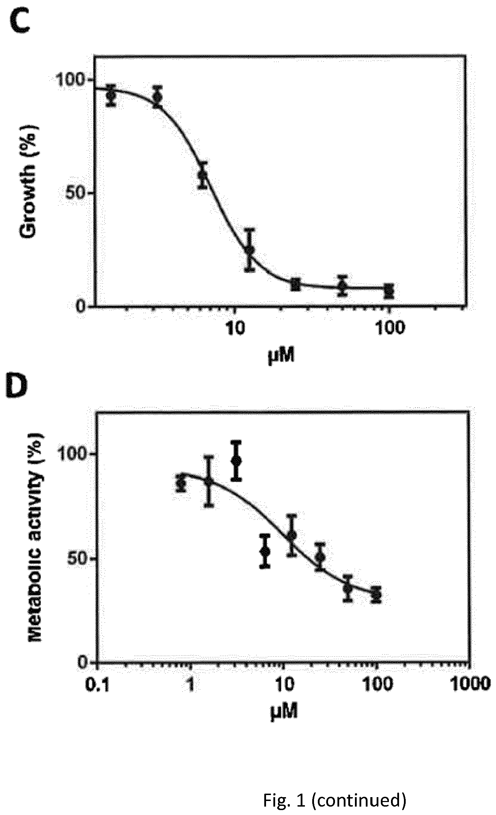

Fig. 1 shows that DHF interferes with planktonic and biofilm cells of Candida spp. in vitro. (A) 3,6-DHF inhibits growth of planktonic *C. albicans* (MIC₅₀ = 9.5 ± 0.1 µM) and (B) *C. albicans* biofilm formation (BIC₅₀ = 55.2 ± 8.1 µM). (C) DHF is active against planktonic *C. glabrata* (MIC₅₀ = 7.9 ± 1.0 µM) and (D) *C. glabrata* biofilm cells (BIC₅₀ = 23.4 ± 9.7 µM). Untreated controls were set to 100%. For dose-response data, sigmoidal curves were generated using non-linear regression and the ICso values were derived from the whole dose-response curves. Data represent means ± s.e.m of at least 3 independent experiments. Data analyzed compared to the untreated control using one-way ANOVA and corrected for multiple comparison using a Bonferroni post-hoc test.

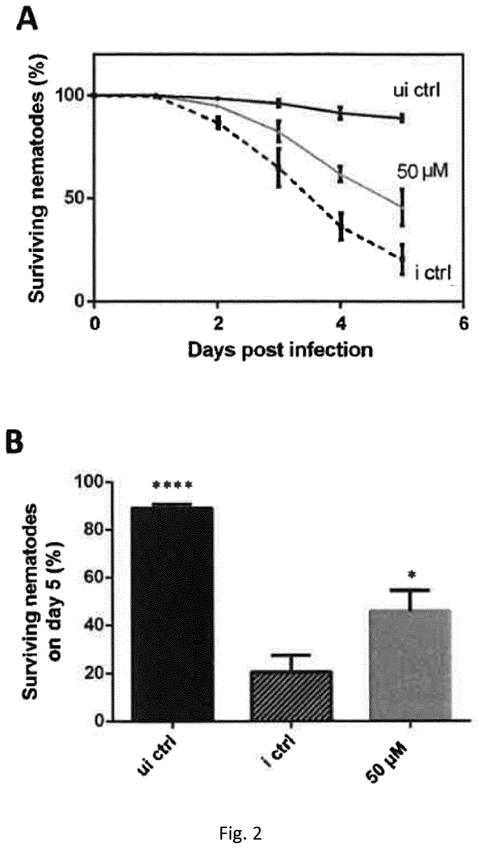

Fig. 2 shows that DHF confers prolonged protection against *C. albicans* infection in vivo. Survival curves of non-infected (ui ctrl) and infected (i ctrl) nematodes treated or not with DHF are shown in (A), and the survival of living nematodes on day 5 is shown in (B). Synchronized C. elegans Aglp-4 1sek-1 larvae were infected and treated as described in Breger J et al.). At least 3 wells for each condition were prepared. Worms were treated with 50 µM DHF or the corresponding volume of solvent (i ctrl) immediately after infection (single application). Worm survival was monitored daily over the period of 5 days. Additionally, the survival of non-infected worms (ui ctrl) was monitored as a control. Data show means ± SEM of 4 independent experiments. In (B), worm survival is expressed as the percentage of viability at day 5 compared to day 0, with data analyzed compared to i ctrl using one-way ANOVA and corrected for multiple comparison using a Bonferroni post-hoc test. * p<0.05, **** p<0.0001.

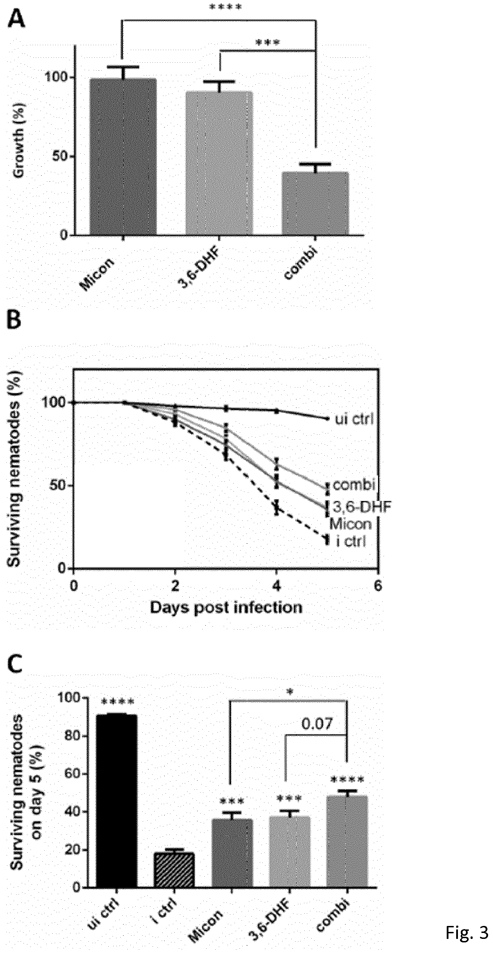

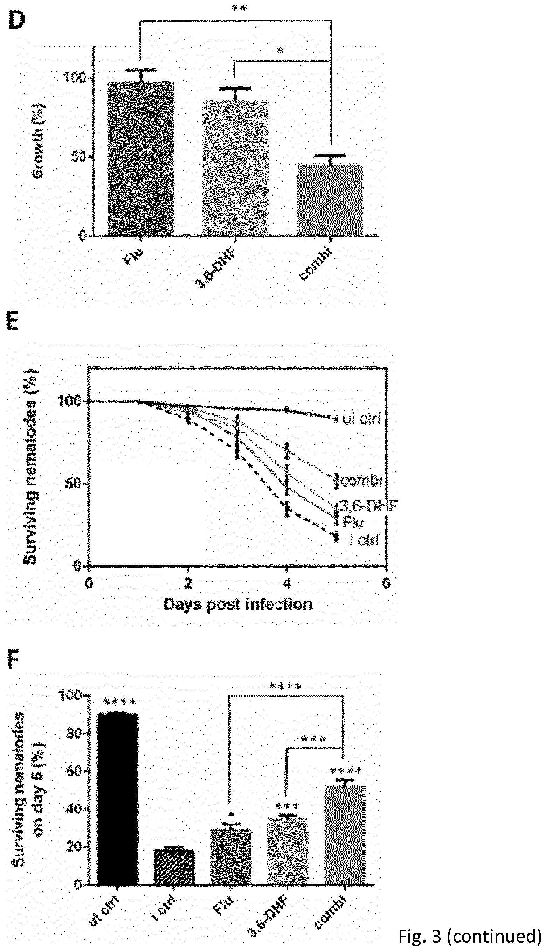

Fig. 3 shows that 3,6-DHF enhances the activity of azoles against *C. albicans* in vitro and in vivo. (A, D) Checkerboard assays were performed and a representative combination (0.4 µM miconazole, 3.1 µM 3,6-DHF in (A); 0.5 µM fluconazole, 3.1 µM 3,6-DH F in (D)) resulting in growth inhibition is shown. (B, C, E, F) *C. elegans* infection experiments were performed as described previously. Worms were treated with either 25 µM 3,6-DHF or 0.125 µM miconazole (B, C) / 0.5 µM fluconazole (E, F) alone or in combination or with the corresponding volume of solvent (i ctrl) immediately after infection (single application). Worm survival was monitored daily over the period of five days. The survival of non-infected worms (ui ctrl) was monitored as a control. Survival curves for the combination of miconazole with 3,6-DHF are shown in (B), and the survival of living nematodes on day 5 is shown in (C). Survival curves for the combination of fluconazole with 3,6-DHF are shown in (E), and the survival of living nematodes on day 5 is shown in (F). In (C, F), worm survival is expressed as the percentage of viability at day 5 compared to day 0 and asterisk above each bar indicate the significance level compared to i ctrl. Data show means ± s.e.m. of at least 3 independent experiments. Data were analyzed using one-way ANOVA and corrected for multiple comparison using a Bonferroni post-hoc test. ns: not significant, * p<0.05, ** p<0.01, *** p<0.001, **** p<0.0001. Micon, miconazole; Flu, fluconazole.

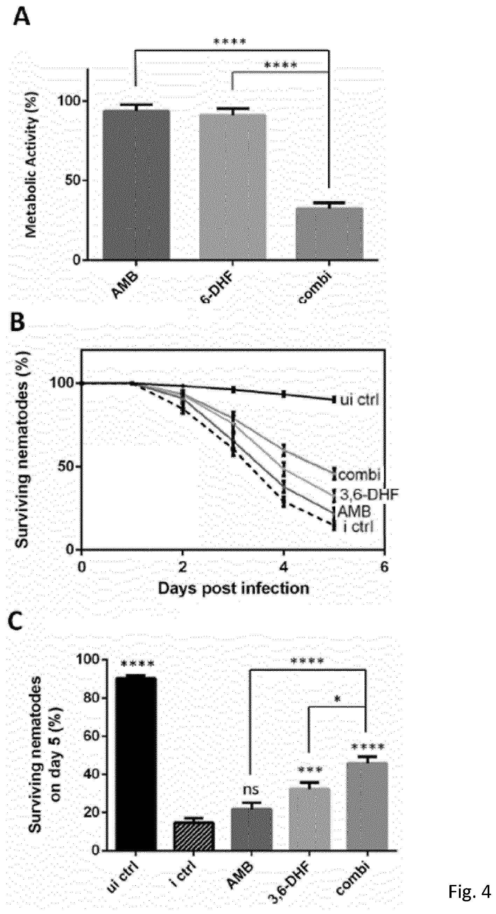

Fig. 4 shows that 3,6-DHF potentiates the antifungal activity of AMB against *C. albicans* in vitro and in vivo. (A) 3,6-DHF acts synergistically with AMB on *C. albicans* biofilms in vitro. Checkerboard assays were performed and biofilm inhibition of substances alone and combinations were measured with CTB staining. A representative combination (0.16 µM AMB, 25µM 3,6-DHF) resulting in synergism is shown in (A). (B, C) 3,6-DHF enhances the antifungal activity of AMB against *C. albicans* in vivo. Survival curves of non-infected (ui ctrl) and infected (i ctrl) nematodes treated or not with 3,6-DHF or AMB alone or in combination are shown in (B), and the survival of living nematodes on day 5 is shown in (D). Synchronized *C. elegans* Aglp-4 Asek-1 larvae were infected as described in Delattin N, et al. (Antimicrob Chemother. 69(2014): 1035-1044). Worms were treated with either 25 µM 3,6-DHF or 0.5 µM AMB alone or in combination, or with the corresponding volume of solvent (i ctrl) immediately after infection (single application). Worm survival was monitored daily over the period of five days. Additionally, the survival of non-infected worms (ui ctrl) was monitored as a control. In (C), worm survival is expressed as the percentage of viability at day 5 compared to day 0 and asterisk above each bar indicates the significance level compared to i ctrl. Data show means ± s.e.m. of at least 3 independent experiments. Data were analyzed using one-way ANOVA and corrected for multiple comparison using a Bonferroni post-hoc test. ns: not significant, * p<0.05, *** p<0.001, **** p<0.0001.

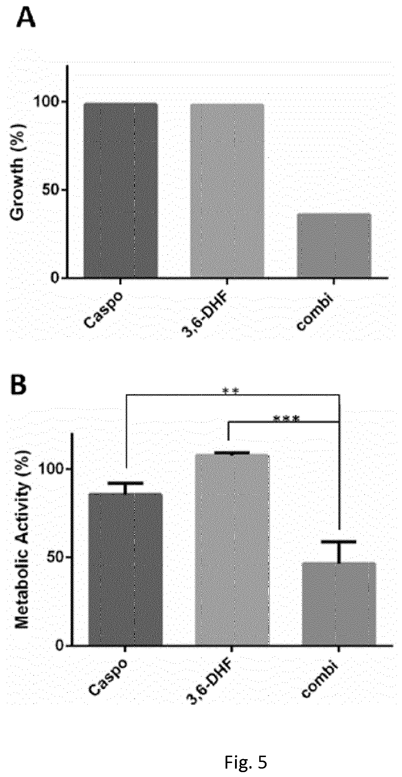

Fig. 5 shows that 3,6-DHF enhances the activity of Caspofungin against *C. albicans.* (A) A representative combination (4.9 nM Caspo, 5 µM 3,6-DHF resulting in growth inhibition of planktonic cells is shown. Preliminary data. (B) Checkerboard assays were performed and biofilm inhibition of substances alone and combinations were measured with CTB staining. A representative combination (39 nM Caspo, 3.1µM 3,6-DHF) resulting in reduced biofilm formation is shown. Data show means ± s.e.m. of at least 3 independent experiments. Data were analyzed using one-way ANOVA and corrected for multiple comparison using a Bonferroni post-hoc test. ** p<0.01, *** p<0.001.

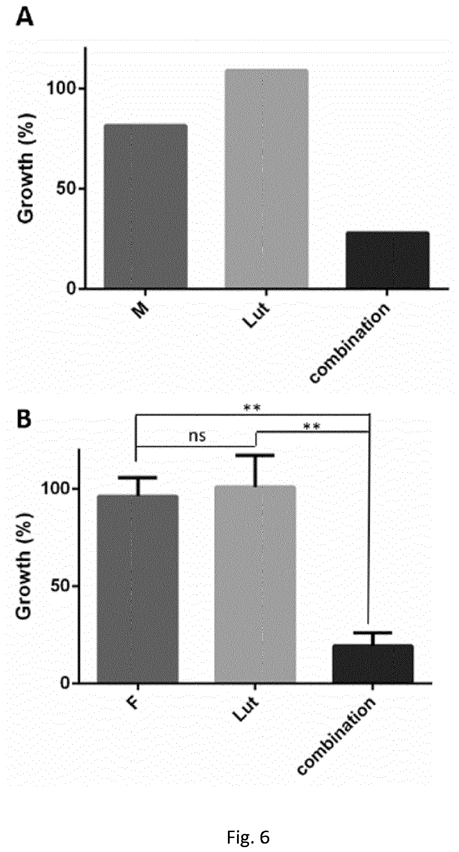

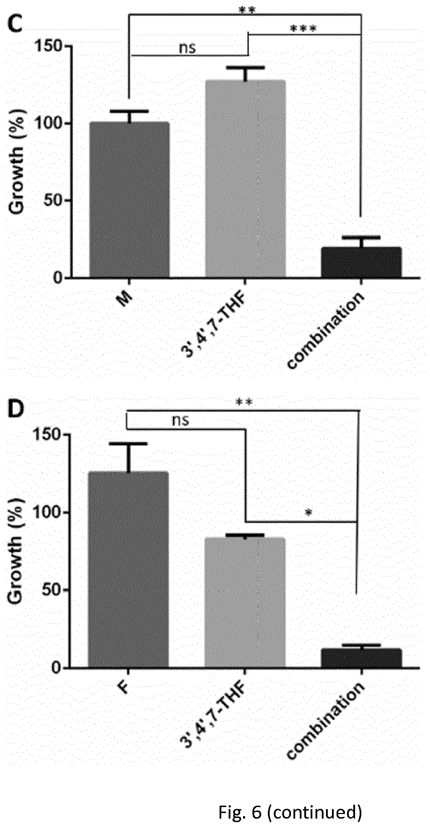

Fig. 6 shows that polyhydroxylated flavones enhance the antifungal activity of azole antifungals. In vitro antifungal acitivity of luteolin (Lut; 3',4',5,7-tetrahydroxyflavone) or 3',4',7-trihydroxyflavone (3',4',7-THF) in combination with miconazole (M) or fluconazole (F) was analyzed by monitoring growth of yeast cells via OD₄₉₀ measurement, and the untreated control was set to 100%. (A, C) In vitro antifungal activity of Lut or 3',4',7-THF in combination with M. *C. albicans* cells were either treated with 0.1 µM M or 12.5 µM (A)/ 6.25 µM (B) flavone alone or in combination. (B, D) In vitro antifungal activity of Lut or 3',4',7-THF in combination with F. *C. albicans* cells were either treated with 1 µM F or 12.5 µM (A)/25 µM (B) flavone alone or in combination. Data represent means ± SEM of at least 3 independent experiments. (A) shows preliminary data of two independent experiments. ns: not significant; * p<0.05; ** p<0.01; *** p<0.001.

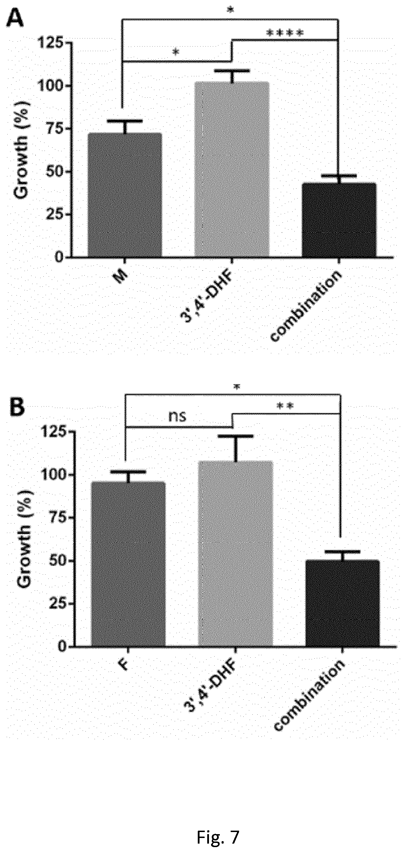

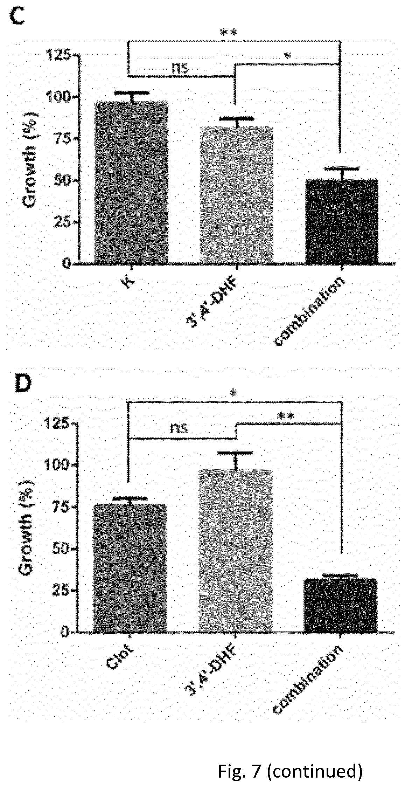

Fig. 7 shows that 3',4'-dihydroxyflavone enhances the antifungal activity of antimycotics. In vitro antifungal acitivity of 3',4'-dihydroxyflavone (3',4'-DHF) in combination with miconazole (M), fluconazole (F), ketoconazole (K) and clotrimazole (Clot) against *C. albicans* was analyzed by monitoring growth of yeast cells via OD₄₉₀ measurement, and the untreated control was set to 100%. (A) Cells were either treated with 0.4 µM M or 25 µM 3',4'-DHF alone or in combination. (B) Cells were either treated with 0.5 µM F or 25 µM 3',4'-DHF alone or in combination. (C) Cells were either treated with 1 µM K or 12.5 µM 3',4'-DHF alone or in combination. (D) Cells were either treated with 0.2 µM Clot or 25 µM 3',4'-DHF alone or in combination. (A-D) Data represent means ± SEM of at least 3 independent experiments. ns: not significant, * p < 0.05, ** p < 0.01, **** p < 0.0001.

### DESCRIPTION OF EMBODIMENTS

According to the present invention substituents R₁, R₂, R₃, R₄, R₅, R₆ and R₇ of the flavones of formula (I) may be independently from each other H or OH. In a particular preferred embodiment of the present invention one or more of substituents R₁, R₂, R₃, R₄, R₅, R₆ and R₇ may be OH. Hence, it is preferred that at least one, preferably at least two, more preferably at least three, more preferably at least five, of substituents R₁, R₂, R₃, R₄, R₅, R₆ and R₇ are OH. In a particularly preferred embodiment of the present invention one, two, three, four, five or six substituents are OH. If substituents R₁, R₂, R₃, R₄, R₅, R₆ and R₇ are not OH the substituents are by default H.

Methods for producing and/or isolating the flavones disclosed herein with the aforementioned substituents are well known in the art (Wagner and Farkas (1975) Synthesis of Flavonoids. In: Harborne, Mabry, Mabry (eds.) The Flavonoids, Springer, Boston (MA, USA); Altemimi et al. (2017), Plants 6(4):42).

The flavones of the present invention influence the physiology and viability of fungal cells, so that these flavones may be used for inhibiting or preventing the growth of fungal cells. The flavones of the present invention may be used to control the growth of fungal cells wherever the presence of fungal cells is not desired. For instance, the flavones may be used in liquids, in suspensions, on surfaces or in any other composition. The flavones can also be used to inhibit the growth of fungal cells in food or feed. Also any kind of surface can be treated with the flavones of the present invention. On the other side the flavones of the present invention can also be used therapeutically in humans and animals, preferably mammals, in order to influence the viability and growth of fungal cells. Hence, the flavones of the present invention can be used in preventing and treating diseases caused by or associated with fungal cells.

According to a preferred embodiment of the present invention R₁ and R₂; R₂ and R₅; R₁ and R₃; R₂ and R₄; R₂ and R₃; R₅ and R₆; R₁; R₂; R₃ or R₅ are OH. Thereby it is preferred that the other substituents are H.

According to a further preferred embodiment of the present invention the flavone is selected from the group consisting of 3,6-dihydroxyflavone, 3,3'-dihydroxyflavone, 6,7-dihydroxyflavone, 2',3-dihydroxyflavone, 3,7-dihydroxyflavone, 3',4'-dihydroxyflavone, 3-hydroxyflavone, 6-hydroxyflavone, 7-hydroxyflavone, 3'-hydroxyflavone, 3',4',5,7-tetrahydroxyflavone, 3',4',7-trihydroxyflavone and 3',4'-dihydroxyflavone, whereby 3,6-dihydroxyflavone is particularly preferred.

According to a preferred embodiment of the present invention the non-filamentous fungal cell is of the class Saccharomycetes, preferably of the family Saccharomycetaceae, more preferably of the genus Candida.

The flavones of the present invention can modulate the growth of non-pathogenic as wells as of pathogenic fungal cells. Pathogenic fungal cells are of major importance so that it is particularly preferred that the fungal cell is a pathogenic fungal cell.

The fungal cells to be contacted with the flavones of the present invention may be single cells, cell colonies or biofilm forming cells, whereby planktonic cells and biofilm forming cells are particularly preferred.

According to a preferred embodiment of the present invention the non-filamentous fungal cell is selected from the group consisting of *Candida albicans, Saccharomyces cerevisiae, Candida tropicalis, Candida dubliniensis, Candida parapsilosis, Candida kefyr, Candida guilliermondii, Candida inconspicua, Candida famata, Candida glabrata, Candida krusei, Candida lusitaniae, Candida auris, Cryptococcus neoformans,* and *Cryptococcus gattii.*

It turned surprisingly out that the flavones of the present invention are not only able to influence or prevent the growth of non-filamentous fungal cells but show significant synergistic effects on fungal cells, filamentous as well as non-filamentous fungal cells, when combined with other antimycotic compounds. Hence, it is particularly preferred to use the flavone having formula (I) in combination with at least one further antimycotic compound.

According to a preferred embodiment of the present invention the filamentous fungal cell is selected from the group consisting of *Aspergillus fumigatus, Aspergillus flavus, Aspergillus niger,* and *Aspergillus terreus.*

According to a preferred embodiment of the present invention the antimycotic compound is selected from the group of azoles, echinocandins and polyenes.

It turned out that the flavones of the present invention show particular synergistic effects in regard to the growth and viability of fungal cells when used in combination with azoles, echinocandins and polyenes.

According to a preferred embodiment of the present invention the azole is an imidazole, a triazole or a thiazole.

According to a further preferred embodiment of the present invention the imidazole is selected from the group consisting of bifonazole, butoconazole, clotrimazole, econazole, fenticonazole, isoconazole, ketoconazole, luliconazole, miconazole, omoconazole, oxiconazole, sertaconazole, sulconazole and tioconazole.

According to another preferred embodiment of the present invention the triazole is selected from the group consisting of albaconazole, efinaconazole, epoxiconazole, fluconazole, isavuconazole, itraconazole, posaconazole, propiconazole, ravuconazole, terconazole and voriconazole.

According to a preferred embodiment of the present invention the thiazole is abafungin.

According to another preferred embodiment of the present invention the echinocandin is selected from the group consisting of anidulafungin, caspofungin and micafungin.

According to a further preferred embodiment of the present invention the polyene is selected from the group consisting of amphotericin B, candicidin, filipin, hamycin, natamycin, nystatin and rimocidin.

According to a particularly preferred embodiment of the present invention the antimycotic compound is selected from the group consisting of amphotericin B, miconazole, ketoconazole, fluconazole, clotrimazole and caspofungin.

The at least one flavone of the present invention and optionally the at least one additional antimycotic compound can be used to prevent or inhibit the growth of fungal cells, preferably non-filamentous fungal cells, for various purposes. Hence, the aforementioned compounds can be applied in various ways depending where and how the growth and viability of fungal cells shall be inhibited or prevented. The at least one flavone may be applied on surfaces, added to suspensions and liquids or even incorporated into polymers, for instance.

The effective concentration of the flavones of the present invention is preferably from 1 to 500 µM, more preferably from 2 to 400 µM, more preferably from 5 to 300µM. In particular, at theses concentrations the flavones of the present invention show to be effective against fungal cells. Hence, it is particularly preferred to apply or administer the flavones of the present invention at these concentrations.

In combination with an antimycotic compound, in particular with one or more azoles, the flavones and the further antimycotic compound(s) are preferably applied or administered at a concentration from 0.1 to 100 µM, preferably from 0.2 to 80 µM, more preferably from 0.5 to 50µM.

According to a preferred embodiment of the present invention the flavones of the present invention are combined with antimycotic compounds, preferably azole, in a molar ratio of 2:1 to 100:1 (flavone:antimycotic compound) .

Plants and parts thereof are often affected by fungal cells. Hence, the flavone of the present invention is preferably applied alone or in combination with a further antimycotic compound as defined herein to a plant or parts thereof, in particular fruits or leaves.

According to a preferred embodiment of the present invention the plant is selected from the group consisting of wheat, barley, millet, oat, corn and rice.

Another aspect of the present invention relates to a composition comprising at least one flavone of formula (I) and optionally at least one further antimycotic compound, wherein R₁, R₂, R₃, R₄, R₅, R₆ and R₇ are independently from each other H or OH.

It was surprisingly found that the flavones of the present invention show antimycotic effects and exhibit synergistic effects in combination with other antimycotic compounds on fungal cells, in particular on non-filamentous fungal cells. In addition thereto, it was found that antimycotic compounds which do not show an effect against certain fungal cells exhibit an antimycotic effect when combined with the flavones of the present invention.

According to a preferred embodiment of the present invention at least one, preferably at least two, more perefably one or two, of R₁, R₂, R₃, R₄, R₅, R₆ and R₇ are OH.

In a particularly preferred embodiment of the present invention the substituents R₁ and R₂; R₂ and R₅; R₁ and R₃; R₂ and R₄; R₂ and R₃; R₅ and R₆; R₁; R₂; R₃ or R₅ are OH.

According to a further preferred embodiment of the present invention the flavone is selected from the group consisting of 3,6-Dihydroxyflavone, 3,3'-Dihydroxyflavone, 6,7-Dihydroxyflavone, 2',3-Dihydroxyflavone, 3,7-Dihydroxyflavone, 3',4'-Dihydroxyflavone, 3-Hydroxyflavone, 6-Hhydroxyflavone, 7-Hydroxyflavone, 3'-hydroxyflavone, 3',4',5,7-tetrahydroxyflavone, 3',4',7-trihydroxyflavone and 3',4'-dihydroxyflavone, whereby 3,6-Dihydroxyflavone is particularly preferred.

According to a preferred embodiment of the present invention the antimycotic compound is selected from the group of azoles, echinocandins and polyenes.

According to a preferred embodiment of the present invention the azole is an imidazole, a triazole or a thiazole.

According to a further preferred embodiment of the present invention the imidazole is selected from the group consisting of bifonazole, butoconazole, clotrimazole, econazole, fenticonazole, isoconazole, ketoconazole, luliconazole, miconazole, omoconazole, oxiconazole, sertaconazole, sulconazole and tioconazole.

According to another preferred embodiment of the present invention the triazole is selected from the group consisting of albaconazole, efinaconazole, epoxiconazole, fluconazole, isavuconazole, itraconazole, posaconazole, propiconazole, ravuconazole, terconazole and voriconazole.

According to a preferred embodiment of the present invention the thiazole is abafungin.

According to a further preferred embodiment of the present invention the echinocandin is selected from the group consisting of anidulafungin, caspofungin and micafungin.

According to another preferred embodiment of the present invention the polyene is selected from the group consisting of amphotericin B, candicidin, filipin, hamycin, natamycin, nystatin and rimocidin.

According to a particularly preferred embodiment of the present invention the antimycotic compound is selected from the group consisting of amphotericin B, miconazole, ketoconazole, fluconazole, clotrimazole and caspofungin.

The composition of the present invention may be used for controlling the growth and viability of fungal cells, preferably of non-filamentous fungal cells, in animals, preferably mammals, and humans. Thus, the composition of the present invention may comprise at least one pharmaceutically acceptable excipient which is mainly dependent from the route of administration and well known to a person skilled in the art and commonly used for antimycotic compositions.

Another aspect of the present invention relates to a composition of the present invention or at least one flavone as defined above for the use in the treatment of a fungal infection in a human or animal, preferably mammalian, subject.

It was surprisingly found that the flavone as well as the composition of the present invention can be used as a medicament for treating or preventing fungal infections in humans and animals, preferably mammals.

The at least one flavone of the present invention is administered preferably to the human or animal, preferably mammalian, subject alone or together or subsequently with at least one further antimycotic compound as defined above.

According to a particularly preferred embodiment of the present invention the composition or the flavone and/or the at least one further antimycotic compound are administered orally, topically or intravenously.

The composition as well as the flavone of the present invention can be formulated for any kind of mode of delivery to the patient including oral, topical, by inhalation, intravenous or parenteral administration. Thus, depending upon chosen mode of administration, the composition and the flavone of the present invention can be formulated with common excipients, diluents or carriers, and formed into tablets, capsules, solutions, suspensions, powders, aerosols and the like. Examples of excipients, diluents, and carriers that are suitable for such formulations include buffers, as well as fillers and extenders such as starch, cellulose, sugars, mannitol and silicic derivatives. Binding agents can also be included such as carboxymethyl cellulose, hydroxymethylcellulose, hydroxypropyl methylcellulose and other cellulose derivatives, alginates, gelatin, and polyvinyl-pyrrolidone.

Moisturizing agents can be included such as glycerol, disintegrating agents such as calcium carbonate and sodium bicarbonate. Agents for retarding dissolution can also be included such as paraffin. Resorption accelerators such as quaternary ammonium compounds can also be included. Surface active agents such as cetyl alcohol and glycerol monostearate can be included. Adsorptive carriers such as kaolin and bentonite can be added. Lubricants such as talc, calcium and magnesium stearate, and solid polyethyl glycols can also be included. Preservatives may also be added. The compositions of the invention can also contain thickening agents such as cellulose and/or cellulose derivatives. They may also contain gums such as xanthan, guar or carbo gum or gum arabic, or alternatively polyethylene glycols, bentones and montmorillonites, and the like.

For oral administration, the flavone and optionally the further antimycotic compound may be present as a powder, a granular formulation, a solution, a suspension or an emulsion or may be presented as a bolus, electuary or paste.

Tablets containing the compounds of the present invention can include buffering agents such as calcium carbonate, magnesium oxide and magnesium carbonate. Tablets can also include inactive ingredients such as cellulose, pre-gelatinized starch, silicon dioxide, hydroxy propyl methyl cellulose, magnesium stearate, microcrystalline cellulose, starch, talc, titanium dioxide, benzoic acid, citric acid, corn starch, mineral oil, polypropylene glycol, sodium phosphate, zinc stearate, and the like. Hard or soft gelatin capsules containing the compounds of the present invention can contain inactive ingredients such as gelatin, microcrystalline cellulose, sodium lauryl sulfate, starch, talc, and titanium dioxide and the like, as well as liquid vehicles such as polyethylene glycols (PEGs) and vegetable oil. Moreover, enteric-coated tablets or capsules are also provided and designed to resist disintegration in the stomach and dissolve in the more neutral to alkaline environment of the duodenum. Orally administered therapeutic compounds of the present invention can also be formulated for sustained release.

A sustained-release formulation can be designed to release the compounds of the present invention, for example, in a particular part of the intestinal or respiratory tract, possibly over a period of time. Coatings, envelopes, and protective matrices may be made, for example, from polymeric substances, such as polylactide-glycolates, liposomes, microemulsions, microparticles, nanoparticles, or waxes.

The flavones and optionally the at least one further antimycotic compounds of the present invention can also be formulated as elixirs or solutions for convenient oral administration or as solutions appropriate for parenteral administration, for instance by intramuscular, subcutaneous, intraperitoneal or intravenous routes.

For parenteral administration, e.g. by injection, for example, bolus injection or continuous infusion, the compounds of the present invention may be presented in unit dose form in ampoules, pre-filled syringes, small volume infusion containers or in multi-dose containers. Preservatives can be added to help maintain the shelve life of the dosage form. The compounds of the present invention and other ingredients may form suspensions, solutions, or emulsions in oily or aqueous vehicles, and may contain formulatory agents such as suspending, stabilizing and/or dispersing agents.

It is particularly preferred to administer the flavones and compositions of the present invention topically or to mucosal surfaces such as the vagina, the rectum, eyes, nose and the mouth. For topical administration, the therapeutic agents may be formulated as is known in the art for direct application to a target area. For topical application creams, milks, gels, dispersion or microemulsions, lotions thickened to a greater or lesser extent, impregnated pads, ointments or sticks, aerosol formulations (e.g., sprays or foams), soaps, detergents, lotions or cakes of soap are particularly preferred. Other conventional forms for this purpose include wound dressings, coated bandages or other polymer coverings, ointments, creams, lotions, pastes, jellies, sprays, and aerosols.

Ointments and creams may, for example, be formulated with an aqueous or oily base with the addition of suitable thickening and/or gelling agents. Lotions may be formulated with an aqueous or oily base and will in general also contain one or more emulsifying agents, stabilizing agents, dispersing agents, suspending agents, thickening agents, or coloring agents. Topical applications may also comprise an adjuvant. Compositions for topical application to skin may include an adjuvant, solvent, or co-solvent to assist the flavone and optionally the additional antimycotic compounds with penetrating the outer dermal layers. An exemplary adjuvant, solvent, or co-solvent is dimethyl sulfoxide (DMSO).

The pharmaceutical formulations of the present invention may include, as optional ingredients, pharmaceutically acceptable carriers, diluents, solubilizing or emulsifying agents, and salts of the type that are available in the art. Examples of such substances include normal saline solutions such as physiologically buffered saline solutions and water. Specific non-limiting examples of the carriers and/or diluents that are useful in the pharmaceutical formulations of the present invention include water and physiologically acceptable buffered saline solutions such as phosphate buffered saline solutions pH 7.0-8.0.

Another aspect of the present invention relates to a method for inhibiting or preventing the growth of a fungal cell, preferably a non-filamentous cell, comprising the step of contacting fungal cells with at least one flavone and/or a composition as defined above.

According to a preferred embodiment of the present invention the at least one flavone is contacted together or subsequently with at least one further antimycotic compound as defined above.

### EXAMPLES

### Material & Methods

### Yeast strains, fungal strains and growth conditions

Candida strains were maintained in YPD media (10 g/L Yeast extract, 20 g/L Peptone, 40 g/L Pextrose/glucose). Experiments were performed in YPD media or in PRMI media (Sigma Aldrich, Germany) buffered with MOPS (TCI, Germany or ABCR, Germany) Agar (20 g/L; BD, Germany) was added to prepare solid media. For long time storage yeast strains in YPD were mixed 1:1 with 50% glycerol as antifreeze and stored at -80°C.

### In vitro antifungal susceptibility testing

### Minimal inhibitory concentration

The minimal inhibitory concentration (MIC) was determined following the standard Clinical and Laboratory Standards Institute (CLSI) protocol M27 (Rex JH, and Clinical and Laboratory Standards Institute (2008). Reference method for broth dilution antifungal susceptibility testing of yeasts: approved standard, 3rd ed. National Committee for Clinical Laboratory Standards, Wayne, PA) for yeast strains or the standard CLSI protocol M38 (Clinical and Laboratory Standards Institute (2008). Reference method for broth dilution antifungal susceptibility testing of filamentous fungi. Clinical and Laboratory Standards Institute, Wayne, PA) for filamentous fungi.

For yeast strains media was inoculated with an overnight culture to an OD₆₀₀ of 0.001 (1⁰⁴ cells/mL) . Serial dilutions of substances were added and cells were incubated at 37°C without shaking for 48 ± 1 h.

For filamentous fungi, ½ PDB media was inoculated with respective spores to a concentration of 4x10⁴ spores/mL. Serial dilutions of substances were added and spores were incubated at 22°C without shaking for three to four days.

MIC tests were performed in flat-bottom 96 well plates (Greiner Bio One, Austria) using 100 µL suspension per well sealed with gas-permeable foils. After incubation, OD490 values were measured using 96-well plate reader and growth capacity was analyzed by setting the corresponding control to 100%.

### Biofilm inhibition concentration

The biofilm inhibition concentration (BIC) was determined as described in Delattin N et al. (J Antimicrob Chemother 69(2014): 1035-1044). In short, RPMI medium was inoculated with an overnight culture to an OD (optical density) of 0.1, transferred to a U-bottom 96-well plate (MLS, Belgium) and treated with serial dilutions of substances. After the adherence phase (1 h, 37°C), supernatant was removed, adherent cells were washed with PBS and fresh media (including substances) was added. Biofilms were then allowed to grow for 24 h at 37°C.

Formed biofilms were then analyzed for their metabolic activity. Therefore, *C. albicans* biofilm cells were treated with CTB (cell titer blue®; Promega, Germany), diluted 1:100 in PBS. After incubation for 1 h at 37°C in the dark fluorescence intensity was measured using a 96-well plate reader (Ex: 535, Em: 590, sens 65). For *C. glabrata,* XTT (2H-Tetrazolium, 2,3-bis(2-methoxy-4-nitro-5-sulfophenyl)-5-[(phenylamino)carbonyl]-hydroxide;

Sigma, Germany or ABCR, Germany) was used instead. Thereby, biofilm cells were treated with an XTT solution (0.25 mg/mL XTT + 0.1 µM menadione in PBS) at 37°C in the dark and OD₄₉₀ was measured.

Additionally, biofilm inhibition was determined by plating experiments. Therefore, grown biofilms were washed, biofilm cells were resuspended in PBS + 1% Triton and serial dilutions were plated on YPD agar plates. Plates were incubated at 37°C for one day and CFUs were counted.

As for MIC, biofilm inhibition was analyzed by setting the corresponding control to 100%.

### Biofilm eradication concentration

To determine the biofilm eradication concentration (BEC), biofilms were grown as described above in the absence of substances. Pre-grown biofilms (24 h, 37°C) were washed with PBS before adding fresh media and serial dilutions of substances. After incubation for 24 h at 37°C, metabolic activity of remaining biofilm cells was analyzed as described above.

### Checkerboard assay (Synergy Testing)

For synergy testing of different combinations, checkerboard assays were performed. Therefore, serial dilutions of two different compounds were combined for MIC, BIC or BEC testing as described above. Antifungal activity of substances alone or in combination was determined by setting the corresponding control to 100%.

### In vivo antifungal susceptibility testing using Caenorhabditis elegans

### C. elegans maintenance and long-time storage

The *C*. *elegans* Δglp-4 Δsek-1 strain (generously provided by Valerie Defraine, Centre of Microbial and Plant Genetics, KU Leuven, Belgium) was used for in vivo testing of antifungal activity. Worms were maintained on NGM plates (nematode growth medium; 2.5 g/L Bacto Peptone, 3 g/L NaCl, 17 g/L agar, supplemented with 5 mg/L cholesterol, 1 mM CaCl₂, 1 mM MgSO₄ and 25 mM KPO₄ buffer pH 6.0), seeded with a thin layer of *E. coli* OP50, at 16°C and worms were chunked onto fresh plates every three to four days.

For longtime storage, starved worms were harvested from plates, diluted 1:1 with 50% glycerol as an antifreeze and stored at -80°C.

### Egg collecting and synchronization of C. elegans

Egg collecting was performed by bleaching according to Stiernagle T ("Maintenance of C. elegans". WormBook. doi: 10.1895/wormbook.1.101 (2006)) and Porta-de-la-Riva M et al. JoVE 64(2012): e4019-e4019). In short, adult worms were collected from plates and treated with a bleaching solution (10 mL household bleach + 5 mL 5M NaOH), thereby worms disintegrate and eggs are released. After vigorous shaking for 5 min, M9 medium (3 g/L KH₂PO₄, 6 g/L Na₂HPO₄, 5 g/L NaCl, supplemented with 1.25 mM MgSO4) was added and worms were immediately put on ice. After three washing steps, residual eggs were incubated in M9 medium over night at 16°C on a tube roller. The next day, eggs were collected, transferred onto fresh NGM/OP50 plates and incubated at 25°C for 3-4 days until nematodes have reached the L3/L4 stage prior to infection.

### Infection with C. albicans and efficacy testing

Synchronized (L3/L4 stage) worms were collected, washed three times with M9 media and spotted onto prepared YPD plates with a thin layer of C. albicans SC5314. Nematodes were fed on YPD/C. albicans plates for of 2 h at 25°C. To remove residual yeast on the cuticula of the worms, nematodes were collected and washed several times with MilliQ water using a sterilized membrane (pore size ~ 20 µM). Collected nematodes were then resuspended in growth medium (M9 medium supplemented with 10 µg/mL cholesterol, 100 µg/mL kanamycin and 75 µg/mL ampicillin) and diluted to a concentration of ~ 40 worms/750 µL.

For efficacy testing of compounds, 750 µL of the worm suspension were transferred into each well of a 24-well plate and substances were added at indicated concentrations (1% DMSO). Nematodes were immediately counted (t0) and survival was monitored over five days post infection by counting living worms.

### Data analysis

Presented data represent mean ± s.e.m of at least three independent experiments. For dose-response experiments, sigmoidal curves were generated using non-linear regression (formula: Y=Bottom + (Top-Bottom)/(1+10^((LogIC50-X)*HillSlope))) and IC50 values were derived from the whole dose-response curves. Data were analyzed by one-way ANOVA and corrected for multiple comparison using a Bonferroni post-hoc test and a confidence level of 0.05. All data were analyzed using GraphPad Prism 6.

### Example 1: Antimycotic effect of flavones

Different flavones were randomly chosen and tested for their effects on planktonic cells and biofilms of *C. albicans* and *C. glabrata* to determine corresponding IC50 values (the concentration that is required to reduce growth (minimal inhibitory concentration = MIC) or biofilm formation (biofilm inhibitory concentration = BIC) to 50% compared to the untreated control).

The antifungal activities of the tested compounds differed greatly depending on the type and respective position of the substitution(s) (see Table 1).

**Table 1. Summary of antifungal susceptibility testing of selected compounds in C. albicans and C. glabrata.**

| | *C. albicans* | | *C. glabrata* | |
|---|---|---|---|---|
| | MIC₅₀ (µM) | BIC₅₀ (µM) | MIC₅₀ (µM) | BIC₅₀ (µM) |
| 3,6-dihydroxyflavone | 9.5±0.1 | 55.2±8.1 | 7.9±1.0 | 23.4±9.7 |
| 3,3'-dihydroxyflavone | 254.4±68.5 | n.d. | 5.9±0.1 | 21.5±3.6 |
| 6,7-dihydroxyflavone | n.d. | n.d. | 15.7±4.1 | 41.7±9.1 |
| 2',3-dihydroxyflavone | n.d. | n.d. | 53.9±2.1 | 123.5±34.7 |
| 3,7-dihydroxyflavone | n.d. | n.d. | n.d. | 62.5±26.0 |
| 3',4'-dihydroxyflavone | n.d. | 146.9±17.3 | n.d. | n.d. |
| 3-hydroxyflavone | n.d. | n.d. | 6.2±0.2 | n.d. |
| 6-hydroxyflavone | n.d. | n.d. | n.d. | 128.8±14.2 |
| 7-hydroxyflavone | n.d. | n.d. | n.d. | 17.4±7.0 |
| 3'-hydroxyflavone | n.d. | n.d. | n.d. | 26.7±3.0 |
| 4',5-dihydroxyflavone | >400 | >400 | >400 | >400 |
| 5,7-dihydroxyflavone | >400 | >400 | >400 | >400 |

| | | | | |
|---|---|---|---|---|
| n.d. - not determined Activities of flavones on planktonic and biofilm cells of *C. albicans* and *C. glabrata* were determined as described in the CLSI protocol M27-A3 (M27-A3; ISBN 1-56238-666-2; 2008; Vol. 28 No. 14) and in Delattin N, et al. (J Antimicrob Chemother. 69(2014): 1035-1044). | | | | |

These results show that not all flavones exhibit antimycotic properties since some substances were not able to reduce growth or biofilm formation of either of both Candida spp. beneath 50% (compared to the untreated control) in the used setup (see e.g. 4',5-dihydroxyflavone and 5,7-dihydroxyflavone). The flavones of the present invention showed antimycotic effects in specific settings, e.g. against biofilms and fungal cells in suspension. However, some flavones seemed to show slightly better effects on fungal cells within biofilms compared to fungal cells in suspension (planktonic cells). In particular 3,6-dihydroxyflavone (DHF) turned out to exhibit outstanding antimycotic effects on fungal cells within a biofilm as well as in suspension (see Table 1 and Fig. 1) .

To verify the antifungal potential of DHF in vivo, the well-established *C. elegans* infection model (Breger J, et al. (2007) PLOS Pathog. 3(2): e18.) was used. Therefore, synchronized L3/L4 larvae were infected by feeding with *C. albicans* SC5314 and treatment was started right after infection. Survival of infected as well as non-infected nematodes was monitored daily over the time period of 5 days (Fig. 2A). DHF was able to counteract the infection of *C. albicans* in vivo, reflected in the improved survival of treated worms compared to the untreated infection control. For instance, at day 5 post infection, 45.7% of the worms treated with 50 µM DHF were still alive compared to only 20.5% in the infected control (Fig. 2B).

### Example 2: Flavones potentiate the antimycotic effect of anitmycotics

In example 1 the antimycotic effect of flavones according to the present invention, like 3,6-DHF, is described. In this example it was examined whether the flavones of the present invention exhibit a potentiating effect in combination with antimycotics of other substance classes like azoles *in vitro* and *in vivo* (see Fig. 3).

Furthermore, the results show that 3,6-DHF exhibits a potentiating effect also in combination with other commercially available antimycotics, for example Amphotericin B (AMB), as a representative of the polyene class, or caspofungin (Caspo), as a representative of the echinocandin class (Figs. 4 and 5).

As shown in Figs. 6 and 7 also the flavones 3',4',5,7-tetrahydroxyflavone, 3',4',7-trihydroxyflavone and 3',4'-dihydroxyflavone show a synergistic antimycotic effect in combination with other known antimycotics.

## Claims

1. Use of a flavone of formula (I) 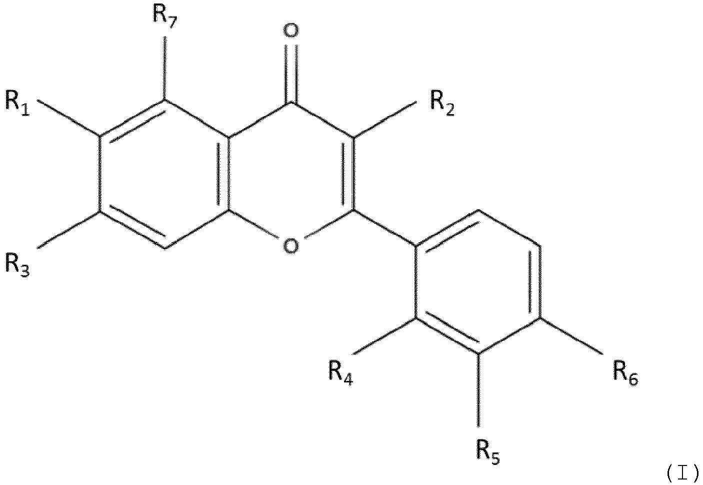 for inhibiting or preventing the growth of a non-filamentous fungal cell, wherein R₁, R₂, R₃, R₄, R₅, R₆ and R₇ are independently from each other H or OH.

2. Use according to claim 1, wherein the flavone is selected from the group consisting of 3,6-dihydroxyflavone, 3,3'-dihydroxyflavone, 6,7-dihydroxyflavone, 2',3-dihydroxyflavone, 3,7-dihydroxyflavone, 3',4'-dihydroxyflavone, 3-hydroxyflavone, 6-hydroxyflavone, 7-hydroxyflavone, 3'-hydroxyflavone, 3',4',5,7-tetrahydroxyflavone, 3',4',7-trihydroxyflavone and 3' ,4'-dihydroxyflavone.

3. Use according to claim 1 or 2, wherein the non-filamentous fungal cell is a biofilm forming fungal cell.

4. Use according to any one of claims 1 to 3, wherein the non-filamentous fungal cell is selected from the group consisting of *Candida albicans, Saccharomyces cerevisiae, Candida tropicalis, Candida dubliniensis, Candida parapsilosis, Candida kefyr, Candida guilliermondii, Candida inconspicua, Candida famata, Candida glabrata, Candida krusei, Candida lusitaniae, Candida auris, Cryptococcus neoformans,* and *Cryptococcus gattii.*

5. Use according to any one of claims 1 to 4, wherein the flavone of formula (I) is used in combination with at least one further antimycotic compound.

6. Use according to claim 5, wherein the antimycotic compound is selected from the group consisting of azoles, echinocandins and polyenes.

7. Use according to any one of claims 1 to 6, wherein the flavone is applied to a plant or parts thereof, in particular fruits or leaves.

8. Composition comprising at least one flavone of formula (I) 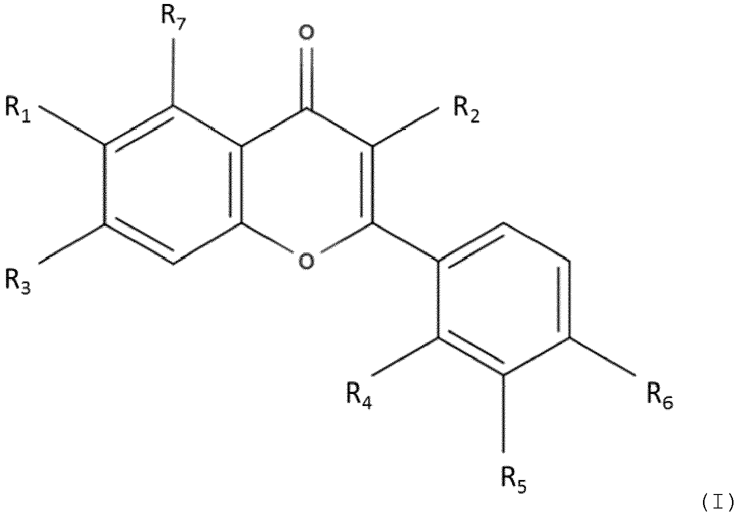 and optionally at least one further antimycotic compound, wherein R₁, R₂, R₃, R₄, R₅, R₆ and R₇ are independently from each other H or OH.

9. Composition according to claim 8, wherein the flavone is selected from the group consisting of 3,6-Dihydroxyflavone, 3,3'-Dihydroxyflavone, 6,7-Dihydroxyflavone, 2',3-Dihydroxyflavone, 3,7-Dihydroxyflavone, 3',4'-Dihydroxyflavone, 3-Hydroxyflavone, 6-Hhydroxyflavone, 7-Hydroxyflavone, 3'-hydroxyflavone, 3',4',5,7-tetrahydroxyflavone, 3',4',7-trihydroxyflavone and 3',4'-dihydroxyflavone.

10. Composition according to claim 8 or 9, wherein the antimycotic compound is selected from the group consisting of azoles, echinocandins and polyenes.

11. Composition according to any one of claims 8 to 10, wherein the composition comprises at least one pharmaceutically acceptable excipient.

12. Composition according to any one of claims 8 to 11 or at least one flavone as defined in claim 1 or 2 for the use in the treatment of a fungal infection in a human or animal, preferably mammalian, subject.

13. Composition or flavone for the use according to claim 12, wherein the at least one flavone is administered to the human or animal, preferably mammalian, subject together or subsequently with at least one further antimycotic compound as defined in claim 6.

14. Composition or flavone for the use according to claim 12 or 13, wherein the composition or the flavone and/or the at least one further antimycotic compound are administered orally, topically or intravenously.

15. Method for inhibiting or preventing the growth of a fungal cell comprising the step of contacting fungal cells with at least one flavone as defined in claim 1 or 2 and/or a composition according to any one of claims 8 to 11
